# FASCICULE DE BREVET EUROPEEN

(11) **EP 1 042 026 B1**
(45) Date de publication et mention de la délivrance du brevet: **19.05.2004**
(21) Numéro de dépôt: 98962516.5
(22) Date de dépôt: 18.12.1998
(51) Int. Cl.: A61M 15/00, B65D 81/32, B01F 13/00, B65D 25/08, A61M 5/24

(54) **DISPOSITIF DE PULVERISATION DE PRODUIT FLUIDE**
SPRÜHVORRICHTUNG FÜR EIN FLÜSSIGES MITTEL
FLUID PRODUCT SPRAYING DEVICE

(30) Priorité: 31.12.1997 FR 9716770
(43) Date de publication de la demande: 11.10.2000
(73) Titulaire: VALOIS S.A.S., 27110 Le Neubourg (FR)
(72) Inventeur: GUIFFRAY, Jean-Louis, F-76000 Rouen (FR)
(74) Mandataire: CAPRI
(86) Numéro de dépôt international: PCT/FR1998/002792
(87) Numéro de publication internationale: WO 1999/034853

(56) Documents cités:
- EP-A- 0 753 354
- WO-A-96/24439
- US-A- 3 946 732
- US-A- 5 380 281
- US-A- 5 569 191

## Description

La présente invention concerne un dispositif de distribution de produit fluide, et plus particulièrement un dispositif permettant de pulvériser en spray une solution formée d'un liquide et d'une poudre reconstituée au dernier moment par le patient.

L'invention concerne plus particulièrement les dispositifs destinés à délivrer un faible nombre de doses et de préférence les unidoses ou bidoses.

Un certain nombre de produits, et plus particulièrement les produits pharmaceutiques, peuvent être très instables en solution dans le temps et/ou peuvent nécessiter des conditions de stockage particulières, qui peuvent être onéreuses. En particulier, ils peuvent être soumis à une oxydation, à une hydrolyse ou à d'autres altérations qui affectent la qualité et l'efficacité du produit. Un moyen d'augmenter la durée de vie et d'éviter les problèmes dûs aux instabilités chimiques de tels composés et/ou aux conditions de stockage, est d'effectuer un stockage sous forme de poudre, puis de reconstituer la solution à l'aide d'un solvant juste avant l'administration du produit. Ce type de dispositif est notamment particulièrement adapté aux traitements par voie nasale.

Les documents EP-0 606 672 et EP-0 562 943 divulguent des dispositifs comportant un réservoir rempli de liquide, un réservoir rempli de poudre et une pompe pour distribuer une solution formée par le mélange des deux produits. Avant utilisation, la poudre est mélangée au liquide puis le liquide est distribué de manière sélective au moyen de la pompe. Ces dispositifs présentent un certain nombre d'inconvénients. De par leur construction, ils ne permettent pas un stockage et/ou un remplissage séparé du réservoir de poudre et du réservoir de liquide, ce qui interdit notamment l'utilisation de la lyophilisation pour le réservoir de poudre. Or, en particulier dans les dispositifs destinés à délivrer un faible nombre de doses, la précision du dosage peut être nettement améliorée en remplissant le réservoir de poudre avec une solution liquide prédosée puis en procédant à une lyophilisation de l'ensemble. Le fait que les deux réservoirs ne peuvent pas être stockés séparément est également un inconvénient du point de vue du stockage du dispositif avant son utilisation.

Certains documents, par exemple US-3 946 732, US-5 380 281 et US-5 569 191, divulguent des dispositifs de distribution tels que des seringues, adaptés à mélanger une poudre et un liquide avant la distribution de ce mélange. Dans ces documents, le processus de mélange est réalisé au moyen d'une aiguille qui vient percer le ou les bouchons obturant les réservoirs pour les mettre en communication. Cette mise en oeuvre présente l'inconvénient que lors du percement de la membrane ou du joint, des particules du matériau constituant cette membrane ou ce joint risquent d'être mélangées au produit fluide à distribuer. Ceci peut polluer ou contaminer le produit ou encore boucher le canal de sortie lors de la distribution.

La présente invention a pour but de fournir un dispositif de distribution de produit fluide qui ne comporte pas les inconvénients précités.

La présente invention a aussi pour but de fournir un dispositif de distribution de produit fluide qui soit simple et peu coûteux à fabriquer et à utiliser.

La présente invention a encore pour but de fournir un tel dispositif destiné à pulvériser une solution réalisée de manière extemporanée, où le réservoir de poudre est réalisé, rempli et/ou stocké de manière séparée du réservoir de liquide. En particulier, la présente invention a pour but de fournir un tel dispositif qui permette de doser le réservoir de poudre par lyophilisation d'une solution liquide prédosée.

La présente invention a aussi pour but de fournir un dispositif de distribution de produit fluide dans lequel la totalité d'une dose de produit est distribuée à chaque actionnement par une bonne pulvérisation, notamment dans le cas des utilisations de type nasal.

La présente invention a de plus pour but de fournir un dispositif de pulvérisation d'un produit fluide obtenu par un mélange préalable de deux produits différents dans lequel le processus de mélange évite tout risque de pollution ou de contamination du produit à pulvériser, et tout risque de bouchage du canal de sortie.

La présente invention a donc pour objet un dispositif de pulvérisation de produit fluide comportant un canal de sortie se terminant par un orifice de pulvérisation, un premier réservoir renfermant un premier produit, un second réservoir renfermant un second produit, lesdits deux réservoirs étant reliés par un passage et étant, avant actionnement du dispositif, séparés de manière étanche par des moyens d'étanchéité disposés dans ledit passage, des moyens de mélange étant prévus pour ouvrir ledit passage et pour mélanger les deux produits avant leur distribution, et des moyens de distribution étant prévus pour distribuer ledit mélange, lesdits moyens de mélange comportant un premier piston disposé coulissant dans l'un des réservoirs pour transférer l'un des produits de son réservoir dans l'autre réservoir, lesdits moyens de distribution comportant un second piston disposé coulissant dans l'autre réservoir pour distribuer le mélange des deux produits à travers le canal de sortie, caractérisé en ce que lesdits moyens d'étanchéité comportent au moins une bille chassable hors du passage, par la pression créée dans l'un des réservoir lors de l'actionnement dudit premier piston.

Avantageusement, lesdits moyens de mélange comportent une tige creuse solidaire du premier piston et adapté à pénétrer dans ledit passage pour chasser la ou les billes et s'étendant après le mélange des deux produits jusqu'au second réservoir, de telle sorte que ladite tige creuse ferme l'extrémité d'entrée du canal de sortie.

De préférence, lesdits moyens de mélange sont actionnés indépendamment desdits moyens de distribution.

Selon une variante préférée, lesdits moyens de distribution sont adaptés à distribuer la totalité du mélange des deux produits en un seul actionnement.

Selon une autre variante, lesdits moyens de distribution comportent ou coopèrent avec des moyens de butée pour fractionner le mélange des deux produits en plusieurs doses, une dose étant distribuée à chaque actionnement.

Selon un mode de réalisation avantageux de l'invention, le dispositif comporte deux sous-ensembles indépendants, le premier sous-ensemble comportant le canal de sortie, le premier réservoir et l'un des pistons, et le second sous-ensemble comportant le second réservoir et l'autre piston, des moyens de connexion étant prévus pour connecter ensemble les deux sous-ensembles.

Avantageusement, les premier et second réservoirs comportent chacun une bille respective chassée lors de l'actionnement des moyens de mélange.

Selon un autre mode de réalisation de l'invention, les moyens de mélange comportent le piston disposé dans le second réservoir, l'actionnement dudit piston créant dans le second réservoir la pression nécessaire pour chasser la bille hors du passage à l'intérieur du premier réservoir et pour transférer le produit contenu dans le second réservoir dans le premier réservoir pour s'y mélanger avec l'autre produit, les moyens de distribution comportant le piston disposé dans le premier réservoir dont l'actionnement assure la distribution du mélange.

De préférence, l'un des produits est un liquide et l'autre produit une poudre, le mélange étant réalisé de manière extemporanée par transfert du produit liquide dans le réservoir contenant la poudre.

Avantageusement, ledit premier réservoir contient un solvant et ledit second réservoir contient une poudre, ou vice-versa.

De préférence, ladite poudre est une solution liquide prédosée puis lyophilisée.

D'autres caractéristiques et avantages de la présente invention apparaîtront au cours de la description détaillée suivante donnée à titre d'exemple non limitatif en regard des dessins joints, sur lesquels :
- la figure 1 est une vue schématique en coupe d'un mode de réalisation avantageux de l'invention, avant assemblage du dispositif,
- la figure 2 est une vue similaire à celle de la figure 1, après assemblage du dispositif et avant mélange des produits,
- la figure 3 est une vue similaire à celle de la figure 2, après mélange des produits et avant distribution du mélange des produits,
- la figure 4 est une vue similaire à celle de la figure 3, après distribution du mélange des produits, et
- la figure 5 est une vue schématique en coupe d'un autre mode de réalisation de l'invention.

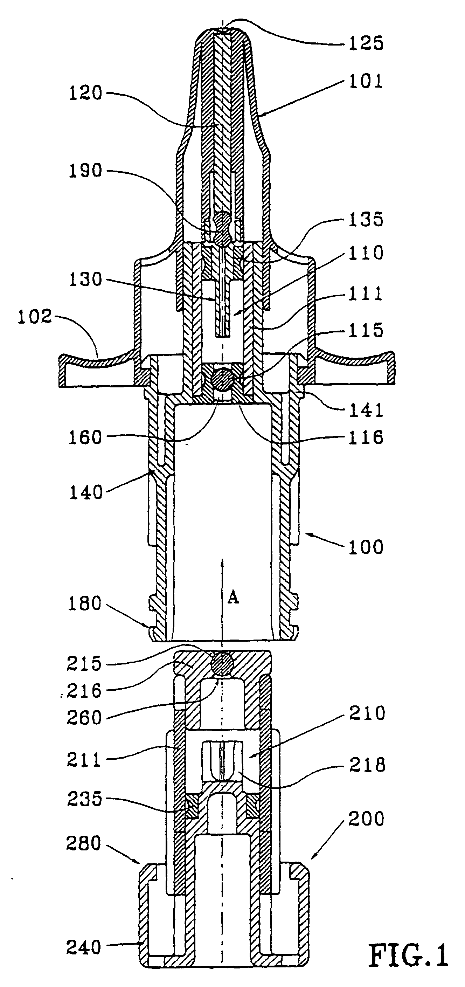

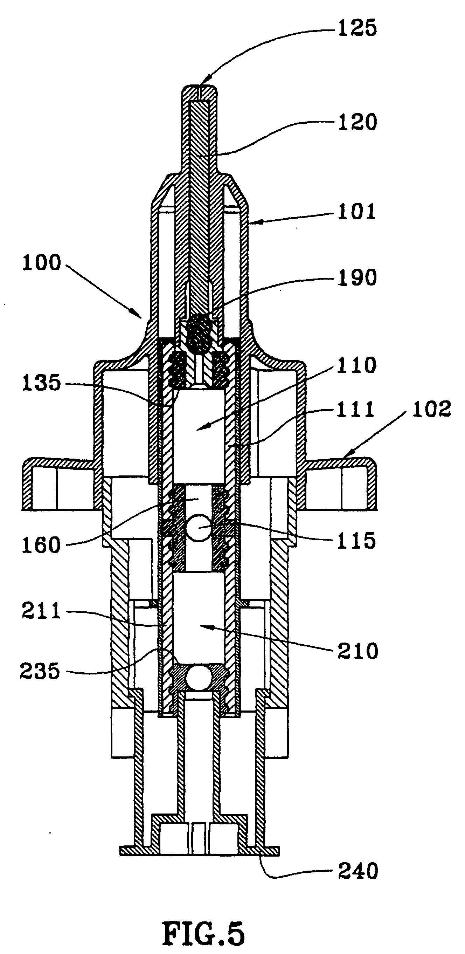

Les figures 1 à 4 représentent un mode de réalisation avantageux, dans lequel le dispositif de distribution est constitué de deux sous-ensembles qui sont séparés et peuvent donc être fabriqués, remplis et stockés séparément pour être assemblés lorsque le dispositif doit être utilisé. Il est entendu que la présente invention peut également s'appliquer à des dispositifs réalisés de manière non séparable, comme représenté par exemple sur la figure 5.

De même, le dispositif décrit sur les dessins s'applique plus particulièrement à une adaptation de type nasal. Le poussoir comporte donc un embout nasal 101 venant dans une narine et une zone d'appui 102 permettant l'actionnement du dispositif. Cette forme extérieure de poussoir nasal est bien connue et ne sera donc pas décrite plus en détail ci-après. Il est entendu que l'invention s'applique à tout type de dispositif et n'est pas limitée aux utilisations de type nasal.

Selon l'invention, le dispositif comporte un premier réservoir 110 qui renferme un premier produit et un second réservoir 210 qui enferme un second produit, les deux produits étant destinés à être mélangés juste avant la distribution pour éviter une altération du mélange. En particulier, l'un des produits est un liquide et l'autre produit est une poudre, de sorte que le mélange est une solution qui peut être finement pulvérisée sous la forme d'un spray à travers l'orifice de sortie 125 du poussoir.

Dans l'exemple de réalisation des figures 1 à 4, le dispositif est constitué de deux sous-ensembles. Le premier sous-ensemble 100 incorpore le premier réservoir 110 et le canal de sortie 120 qui débouche dans l'orifice de sortie 125 et qui est entouré par l'embout nasal 101 se prolongeant jusqu'à la zone d'appui 102 du poussoir. Le canal de sortie 120 est de préférence substantiellement rempli par un gicleur interne qui permet de limiter au maximum le volume mort et d'assurer ainsi une parfaite pulvérisation lors de l'actionnement du dispositif. Avantageusement, ce gicleur interne se prolonge sur une majeure partie de l'embout nasal 101 jusqu'à un obturateur élastique 190 prévu dans le canal de sortie et qui forme clapet de sortie. Le premier réservoir 110 comporte avantageusement un tube en verre 111 qui permet de garantir le stockage du liquide dans le temps. Ce réservoir 110 est d'une part obturé par une paroi ou un élément d'obturation 116 qui comporte un passage 160 dans lequel vient s'insérer un moyen d'étanchéité 115, tel qu'une bille, pour fermer le réservoir 110 après son remplissage. Du côté opposé, selon l'invention, le réservoir comporte un piston 135 qui peut coulisser de manière étanche à l'intérieur du réservoir, ledit piston faisant partie des moyens de mélange utilisés pour réaliser le mélange des deux produits du dispositif comme expliqué ci-après.

Le premier sous-ensemble 100 comporte en outre un corps 140 solidaire du réservoir 110 et qui se connecte au poussoir 101, 102 de telle sorte que le poussoir peut coulisser par rapport audit corps 140, pour ainsi déplacer ledit piston 135 à l'intérieur du réservoir 110. Le corps 140 comporte de préférence des moyens de butée 141 qui empêchent tout déplacement relatif du poussoir par rapport au corps 140 dans une première position, dite position d'attente, et qui permettent ce déplacement dans une seconde position, dite position avant-mélange. En particulier, le déplacement du poussoir vers cette position avant-mélange est réalisé par rotation du poussoir autour du corps 140. De son côté opposé, le corps 140 comporte des moyens de connexion 180 adaptés à réaliser une connexion avec le second sous-ensemble 200 du dispositif qui sera décrit ci-après.

Le second sous-ensemble 200 du dispositif comporte le second réservoir 210 contenant le second produit, de préférence une poudre. Ce second réservoir est obturé d'un côté par une paroi ou élément d'obturation 216 qui comporte un passage 260 obturé par un élément d'étanchéité 215, de préférence une bille. Du côté opposé, le réservoir 210 comporte un piston 235 qui peut coulisser de manière étanche dans le second réservoir 210 pour distribuer le produit contenu dans celui-ci. Ce piston 235 est sollicité en déplacement par un corps 240 qui est actionné par l'utilisateur pour distribuer le produit. Ce corps 240 peut donc coulisser par rapport audit premier réservoir 210 et comporte avantageusement les moyens de connexion 280 pour se connecter aux moyens de connexion 180 du premier sous-ensemble 100. Cette connexion des deux sous-ensembles se fait avantageusement par encliquetage.

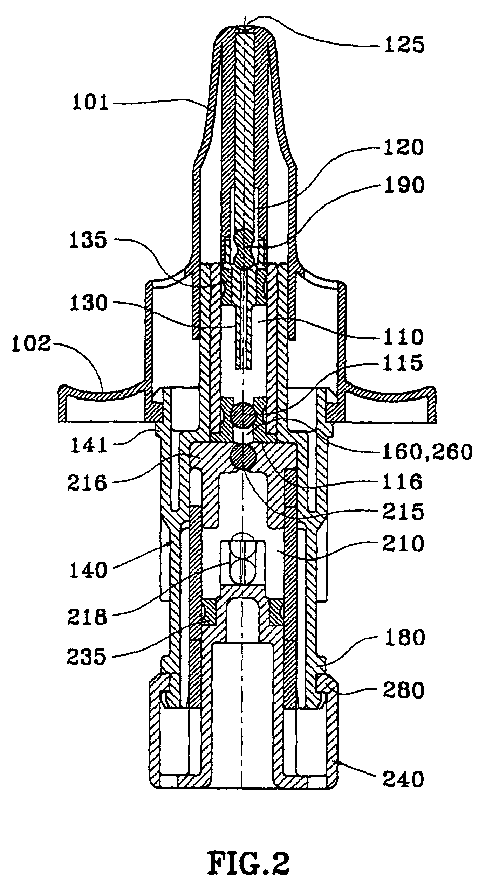

En référence aux figures 1 et 2, l'assemblage des deux sous-ensembles est représenté de manière schématique. Ainsi, le premier réservoir 110 est rempli avec un produit liquide puis obturé par l'élément d'obturation 116 et une bille 115 alors que de manière séparée, le second réservoir 210 est rempli avec une poudre puis obturé par l'élément d'obturation 216 et une bille 215, de sorte que les deux sous-ensembles peuvent être stockés séparément. Les moyens d'obturation respectifs 115, 116 et 215, 216 peuvent bien sûr être réalisés d'une quelconque manière appropriée. Pour l'assemblage, le second sous-ensemble 200 est inséré dans le sens de la flèche A à l'intérieur de la jupe du corps 140 du premier sous-ensemble 100. Lorsque la paroi ou élément d'obturation 216 du second sous-ensemble 200 vient en butée contre la paroi ou élément d'obturation 116 du premier sous-ensemble 100, les moyens de connexion 180 et 280 des deux sous-ensembles s'encliquettent pour solidariser les deux sous-ensembles comme représenté sur la figure 2. D'autre part, les passages 160 et 260 sont alignés pour former un passage reliant le premier réservoir 110 au second réservoir 210, ledit passage étant obturé par les deux moyens d'étanchéité, à savoir les deux billes 115 et 215 dans l'exemple représenté.

Comme visible sur les figures, dans le mode de réalisation préféré de l'invention, le second réservoir 210 comporte des moyens de réception 218 pour recevoir lesdits moyens d'étanchéité 115 et 215 après qu'ils ont été ouverts, c'est-à-dire déplacés de leur position d'obturation par les moyens de mélange. Sur la figure 2, le dispositif est donc représenté dans sa position d'attente, les deux sous-ensembles étant connectés l'un à l'autre.

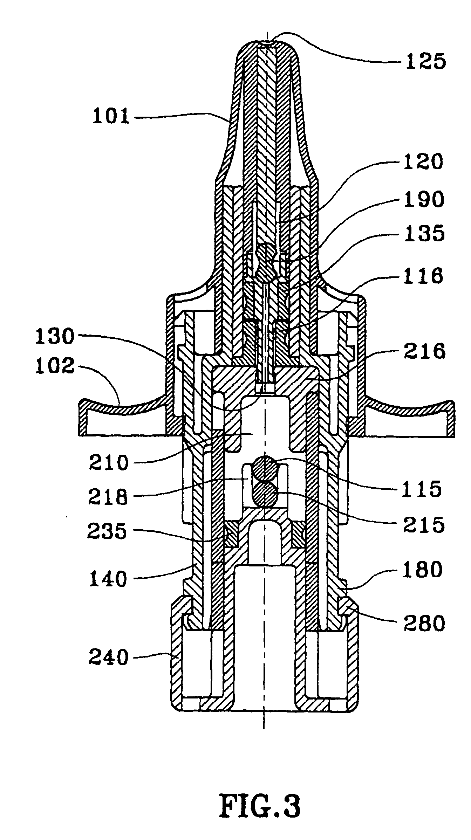

Juste avant que l'utilisateur souhaite utiliser le dispositif, il procède au mélange des deux produits. Pour ce faire, les moyens de mélange prévus dans le premier sous-ensemble 100 comportent de préférence une tige creuse 130 qui forme une partie du canal de sortie 120 et qui est solidaire du premier piston 135 coulissant dans le premier réservoir 110. Avantageusement, cette tige creuse 130 est adaptée à pénétrer dans le passage 160, 260 formé entre les deux réservoirs. Elle peut être adaptée à chasser les moyens d'étanchéité 115 et 215 hors leur position d'obturation, mais cette variante de réalisation n'est pas couverte par l'invention. Ainsi, lorsque l'utilisateur souhaite réaliser le mélange, il fait tourner le poussoir autour du corps 140 du dispositif vers la position avant-mélange, puis il exerce une pression sur le poussoir pour réaliser un déplacement relatif du corps 140 par rapport au poussoir. Lors de ce déplacement, le piston 135 coulisse dans le premier réservoir 110 et la bille 115 puis le bille 215 sont chassées hors de leur position d'obturation ouvrant ainsi le passage 160, 260 entre les deux réservoirs. Le passage étant ouvert, le liquide contenu dans le premier réservoir 110 est transféré dans le second réservoir 210 pour se mélanger à la poudre qui y est contenue. Après achèvement du déplacement relatif entre le poussoir et le corps 140, la totalité du produit liquide a été transférée dans le second réservoir et le dispositif est dans une position, dite position avant-distribution. La tige 130 s'étend de préférence jusqu'à l'entrée du second réservoir 210 pour former ainsi l'entrée du canal de sortie 120. Comme représenté sur la figure 3, dans la position avant-distribution où le mélange est réalisé, les deux billes 115 et 215 sont reçues dans les moyens de réception 218 du second réservoir 210 et l'extrémité de la tige creuse 130 est située à proximité immédiate de l'entrée du réservoir 210. Ainsi, le volume mort est limité au maximum, en particulier du fait que le premier réservoir 110 a complètement disparu dans la position représentée sur la figure 3. De plus, l'utilisation de moyens d'étanchéité, tels que des billes, évite les inconvénients existants dans les dispositifs antérieurs qui utilisaient des membranes perçables par une aiguille. En variante, on peut se passer de la tige creuse 130. Selon l'invention, les billes sont chassées hors du passage 160, 260 par la pression créée dans le premier réservoir 110 lors de l'actionnement du premier piston 135.

Dans la position avant-distribution représentée sur la figure 3, le dispositif est prêt à être utilisé. lorsque l'utilisateur souhaite distribuer le produit contenu dans le second réservoir 210, il exerce une pression sur le poussoir et le corps 240 de sorte qu'un déplacement axial relatif se produit entre le corps 240 et le corps 140, entraînant le piston 235 à coulisser de manière étanche à l'intérieur du second réservoir 210, expulsant ainsi le contenu dudit réservoir. De préférence, l'utilisateur doit exercer une rotation du corps 240 par rapport au corps 140 pour déplacer les moyens de connexion 180, 280 d'une position où le déplacement axial est empêché vers une position où les moyens de connexion coopèrent de telle manière à permettre un déplacement axial des deux corps 140 et 240 l'un par rapport à l'autre.

Lorsque le dispositif est un unidose, un seul actionnement expulse la totalité du contenu du second réservoir 210. Toutefois, on peut également prévoir deux ou plusieurs doses en prévoyant des moyens de butée (non représentés) disposés par exemple sur le corps 140 pour former des arrêts de dose, le contenu du réservoir 210 étant ainsi fractionné en plusieurs doses. Pour surmonter lesdits moyens de butée et donc délivrer les doses ultérieures, le corps 240 doit avantageusement également être tourné par rapport au corps 140 pour permettre une continuation de la course du piston 235 dans le second réservoir 210.

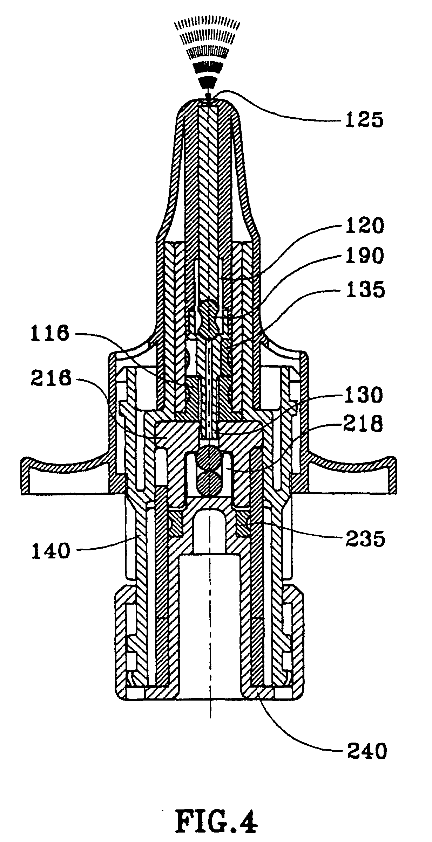

Dans la position de fin d'expulsion représentée sur la figure 4, on constate que le second réservoir 210 a également complètement disparu, l'entrée de la tige creuse 130 étant située à proximité immédiate des moyens d'étanchéité 115, 215 reçus dans les moyens de réception 218 du second réservoir, de sorte que le volume mort est quasi-nul, garantissant l'expulsion de la quasi-totalité du produit contenu dans le second réservoir.

La description ci-dessus a été réalisée en référence à un mode de réalisation avantageux, dans lequel le dispositif est constitué de deux sous-ensembles séparés. Toutefois, il est également envisageable de réaliser le dispositif d'un seul tenant. Dans ce cas, un seul moyen d'étanchéité suffit dans le passage reliant les deux réservoirs, mais le fonctionnement général du dispositif est similaire à celui décrit précédemment.

La figure 5 représente une telle variante de réalisation. Le dispositif comporte deux réservoirs 110 et 210 contenant chacun un piston 135 et 235 et séparés par un passage 160 obturé de manière étanche par une bille 115. Dans cette variante, le réservoir "supérieur " 110 contient avantageusement une poudre et le réservoir "inférieur" contient avantageusement un solvant (les termes supérieur et inférieur se réfèrent à la position du dispositif représenté sur la figure 5). Les moyens de mélange comportent dans ce cas le piston 235 actionné par pression sur le corps 240. Cet actionnement crée une surpression dans le second réservoir 210 qui chasse la bille 115 hors du passage 160 dans le premier réservoir 110. La course du piston 235 transfère ensuite le liquide vers le premier réservoir 110 où le mélange des deux produits se réalise. Ce mélange est ensuite distribué par actionnement du premier piston 135. Dans cette variante, les deux réservoirs font partie du même sous-ensemble et sont donc conditionnés et stockés ensemble. L'avantage de cette variante est sa simplicité, le faible nombre de pièces constitutives et sa fiabilité d'actionnement.

Toutefois, le mode de réalisation des figures 1 à 4 constitué de deux sous-ensembles présente aussi des avantages importants. Ainsi, la fabrication, le remplissage et/ou le stockage des deux sous-ensembles peuvent être réalisés de manière séparée. Ceci permet un gain de place, facilite grandement le remplissage des deux réservoirs et surtout permet d'utiliser la lyophilisation pour former la poudre dans le second réservoir 210. En effet, afin d'améliorer la précision du dosage, le second réservoir 210 est de préférence rempli avec une solution liquide prédosée, puis le bouchon d'obturation 216 pourvu de sa bille 215 est prépositionné sur ledit réservoir 210, sans le fermer. Ensuite la lyophilisation est réalisée laissant ainsi une quantité dosée de poudre à l'intérieur du réservoir 210, et enfin le bouchon est enfoncé pour obturer de manière étanche ledit réservoir 210. Il est évident que cette forme de réalisation préférée utilisant la lyophilisation n'est pas indispensable et que le second réservoir 210 peut être prérempli avec une quantité souhaitée de poudre.

Un autre avantage du dispositif selon les deux modes de réalisation décrits est qu'il permet de réaliser un dispositif compact qui est sûr pour l'utilisateur en empêchant tout actionnement non souhaité du fait qu'il rend indispensable une rotation relative du poussoir par rapport à un premier corps (par exemple, le corps 140 dans l'exemple des figures 1 à 4) pour réaliser le mélange, et dudit premier corps par rapport à un second corps (par exemple, le corps 240 dans l'exemple des figures 1 à 4) pour distribuer ledit mélange. Il en est de même lorsque le contenu du réservoir à distribuer est fractionné en plusieurs doses. Toute utilisation intempestive ou accidentelle est donc empêchée.

D'autres variantes du dispositif sont envisageables sans sortir du cadre de la présente invention. Ainsi, l'obturateur 190 prévu dans le canal de sortie 120 peut être réalisé de manière quelconque. Dans l'exemple représenté sur les figures, il est constitué d'une pièce en élastomère ou en caoutchouc qui se déforme sous la pression exercée par le produit. L'obturateur pourrait aussi être disposé à proximité immédiate de l'orifice de sortie. De même, la forme extérieure générale du dispositif peut être modifiée. Les moyens de connexion entre le poussoir et le corps 140 et entre le corps 140 et le corps 240 peuvent également être réalisés de manière différente. Avantageusement, il est souhaitable que ces moyens de connexion réalisent cette fonction de sécurité empêchant l'actionnement dans une position angulaire donnée et l'autorisant dans une position angulaire différente. De plus, les moyens de connexion 180 et 280 entre le corps 140 du premier sous-ensemble et le corps 240 du second sous-ensemble peuvent être réalisés de telle sorte qu'en position permettant le déplacement axial relatif de ces deux corps, une petite nervure ne libère ce déplacement axial qu'à partir d'une force prédéterminée exercée sur le dispositif, de sorte que l'énergie est emmagasinée dans la main de l'utilisateur, garantissant une expulsion totale de la dose à chaque actionnement.

## Revendications

1. Dispositif de pulvérisation de produit fluide comportant un canal de sortie (120) se terminant par un orifice de pulvérisation (125), un premier réservoir (110) renfermant un premier produit, un second réservoir (210) renfermant un second produit, lesdits deux réservoirs (110, 210) étant reliés par un passage (160, 260) et étant, avant actionnement du dispositif, séparés de manière étanche par des moyens d'étanchéité (115, 215) disposés dans ledit passage (160, 260), des moyens de mélange (130, 135 ; 235) étant prévus pour ouvrir ledit passage (160, 260) et pour mélanger les deux produits avant leur distribution, et des moyens de distribution (235 ; 135) étant prévus pour distribuer ledit mélange, lesdits moyens de mélange comportant un premier piston (135 ; 235) disposé coulissant dans l'un des réservoirs (110 ; 210) pour transférer l'un des produits de son réservoir (110 ; 210) dans l'autre réservoir (210 ; 110), lesdits moyens de distribution comportant un second piston (235 ; 135) disposé coulissant dans l'autre réservoir (210 ; 110) pour distribuer le mélange des deux produits à travers le canal de sortie (120), **caractérisé en ce que** lesdits moyens d'étanchéité comportent au moins une bille (115, 215) chassable hors du passage (160, 260), par la pression créée dans l'un des réservoir (110 ; 210) lors de l'actionnement dudit premier piston (135 ; 235).

2. Dispositif selon la revendication 1, dans lequel lesdits moyens de mélange comportent une tige creuse (130) solidaire du premier piston (135) et adapté à pénétrer dans ledit passage (160, 260) pour chasser la ou les billes (115, 215), et s'étendant après le mélange des deux produits jusqu'au second réservoir (210), de telle sorte que ladite tige creuse (130) forme l'extrémité d'entrée du canal de sortie (120).

3. Dispositif selon la revendication 1 ou 2, dans lequel lesdits moyens de mélange (130, 135 ; 235) sont actionnés indépendamment desdits moyens de distribution (235 ; 135).

4. Dispositif selon l'une quelconque des revendications 1 à 3, dans lequel lesdits moyens de distribution (235 ; 135) sont adaptés à distribuer la totalité du mélange des deux produits en un seul actionnement.

5. Dispositif selon l'une quelconque des revendications 1 à 3, dans lequel lesdits moyens de distribution (235 ; 135) comportent ou coopèrent avec des moyens de butée pour fractionner le mélange des deux produits en plusieurs doses, une dose étant distribuée à chaque actionnement.

6. Dispositif selon l'une quelconque des revendications précedentes, dans lequel le dispositif comporte deux sous-ensembles (100, 200) indépendants, le premier sous-ensemble (100) comportant le canal de sortie (120), le premier réservoir (110) et l'un des pistons (135), et le second sous-ensemble (200) comportant le second réservoir (210) et l'autre piston (235), des moyens de connexion (180, 280) étant prévus pour connecter ensemble les deux sous-ensembles (100, 200).

7. Dispositif selon la revendication 6, dans lequel les premier et second réservoirs (110, 210) comportent chacun une bille respective (115, 215) chassée lors de l'actionnement des moyens de mélange.

8. Dispositif selon l'une quelconque des revendications précédentes, dans lequel les moyens de mélange comportent le piston (235) disposé dans le second réservoir (210), l'actionnement dudit piston (235) créant dans le second réservoir (210) la pression nécessaire pour chasser la bille (115) hors du passage (160) à l'intérieur du premier réservoir (110) et pour transférer le produit contenu dans le second réservoir (210) dans la premier réservoir (110) pour s'y mélanger avec l'autre produit, les moyens de distribution comportant le piston (135) disposé dans le premier réservoir (110) dont l'actionnement assure la distribution du mélange.

9. Dispositif selon l'une quelconque des revendications précédentes, dans lequel l'un des produits est un liquide et l'autre produit est une poudre, le mélange étant réalisé de manière extemporanée par transfert du produit liquide dans Je réservoir contenant la poudre.

10. Dispositif selon l'une quelconque des revendications précédentes, dans lequel ledit premier réservoir (110) contient un solvant et ledit second réservoir (210) contient une poudre, ou vice-versa.

11. Dispositif selon la revendication 9 ou 10, dans lequel ladite poudre est une solution liquide prédosée puis lyophilisée.

## Patentansprüche

1. Zerstäubungsvorrichtung für ein fluidförmiges Produkt, die einen Ausgangskanal (120), der in einer Zerstäubungsöffnung (125) endet, einen ersten Behälter (110), der ein erstes Produkt enthält, und einen zweiten Behälter (210) umfasst, der ein zweites Produkt enthält, wobei die beiden Behälter (110, 210) durch einen Durchgang (160, 260) verbunden und vor der Betätigung der Vorrichtung in dichter Weise durch Dichteinrichtungen (115, 215) voneinander getrennt sind, die in dem Durchgang (160, 260) angeordnet sind, wobei Mischeinrichtungen (130, 135; 235) vorgesehen sind, um den Durchgang (160, 260) zu öffnen und die beiden Produkte vor ihrer Abgabe miteinander zu vermischen, und wobei weiterhin Abgabeeinrichtungen (235, 135) vorgesehen sind, um diese Mischung abzugeben, wobei die Mischeinrichtungen einen ersten Kolben (135; 235) umfassen, der in gleitender Weise in einem der Behälter (110; 210) angeordnet ist, um das eine der Produkte aus seinem Behälter (110; 210) in den anderen Behälter (210; 110) zu überführen, wobei die Abgabeeinrichtungen einen zweiten Kolben (235; 135) umfassen, der in gleitender Weise in dem anderen Behälter (210; 110) angeordnet ist, um die Mischung der beiden Produkte durch den Ausgangskanal (120) abzugeben, **dadurch gekennzeichnet, daß** die Dichteinrichtungen wenigstens eine Kugel (115, 215) umfassen, die durch den Druck, der in dem einen der Behälter (110; 210) bei der Betätigung des ersten Kolbens (135; 235) erzeugt wird, aus dem Durchgang (160; 260) ausstoßbar ist.

2. Vorrichtung nach Anspruch 1, bei der die Mischeinrichtungen eine hohle Stange (130) umfassen, die mit dem ersten Kolben (135) fest verbunden und geeignet ist, in den Durchgang (160, 260) einzudringen, um die Kugel oder Kugeln (115, 215) auszustoßen, und die sich nach der Vermischung der beiden Produkte bis zum zweiten Behälter (210) derart erstreckt, daß die hohle Stange (130) das Eintrittsende des Ausgangskanals (120) bildet.

3. Vorrichtung nach Anspruch 1 oder 2, bei der die Mischeinrichtungen (130, 135; 235) unabhängig von den Abgabeeinrichtungen (235; 135) betätigt werden.

4. Vorrichtung nach einem der Ansprüche 1 bis 3, bei der die Abgabeeinrichtungen (235; 135) geeignet sind, die gesamte Mischung der beiden Produkte bei einer einzigen Betätigung abzugeben.

5. Vorrichtung nach einem der Ansprüche 1 bis 3, bei der die Abgabeeinrichtungen (235; 135) Anschlagseinrichtungen umfassen oder mit solchen zusammenwirken, um die Mischung der beiden Produkte in mehrere Dosen aufzuteilen, wobei eine Dosis bei jeder Betätigung abgegeben wird.

6. Vorrichtung nach einem der vorhergehenden Ansprüche, bei der die Vorrichtung zwei unabhängige Untereinheiten (100, 200) umfasst, wobei die erste Untereinheit (100) den Ausgangskanal (120), den ersten Behälter (110) und einen der Kolben (135) und die zweite Untereinheit (200) den zweiten Behälter (210) und den anderen Kolben (235) umfasst, und Verbindungseinrichtungen (180, 280) vorgesehen sind, um die beiden Untereinheiten (100, 200) miteinander zu verbinden.

7. Vorrichtung nach Anspruch 6, bei der der erste und der zweite Behälter (110, 210) jeweils eine entsprechende Kugel (115, 215) umfassen, die bei der Betätigung der Mischeinrichtungen ausgestoßen wird.

8. Vorrichtung nach einem der vorhergehenden Ansprüche, bei der die Mischeinrichtungen den Kolben (235) umfassen, der in dem zweiten Behälter (210) angeordnet ist, wobei die Betätigung dieses Kolbens (235) in dem zweiten Behälter (210) den Druck erzeugt, der erforderlich ist, um die Kugel (115) aus dem Durchgang (160) in das Innere des ersten Behälters (110) auszustoßen und das im zweiten Behälter (210) enthaltene Produkt in den ersten Behälter (110) für eine Vermischung mit dem anderen Produkt zu überführen, wobei die Abgabeeinrichtungen den Kolben (135) umfassen, der im ersten Behälter (110) angeordnet ist, und dessen Betätigung die Abgabe der Mischung sicherstellt.

9. Vorrichtung nach einem der vorhergehenden Ansprüche, bei der eines der Produkte eine Flüssigkeit und das andere Produkt ein Pulver ist, wobei die Mischung in momentaner Weise durch die Überführung des flüssigen Produktes in den Behälter realisiert wird, der das Pulver enthält.

10. Vorrichtung nach einem der vorhergehenden Ansprüche, bei der der erste Behälter (110) ein Lösemittel und der zweite Behälter (210) ein Pulver enthält oder umgekehrt.

11. Vorrichtung nach Anspruch 9 oder 10, bei der das Pulver eine vordosierte und dann lyophilisierte flüssige Lösung ist.

## Claims

1. A fluid spray device comprising an outlet channel (120) terminating in a spray orifice (125), a first tank (110) containing a first substance, a second tank (210) containing a second substance, said two tanks (110, 210) being interconnected by a passage (160, 260) and, prior to actuation of the device, being separated from each other in leakproof manner by sealing means (115, 215) disposed in said passage (160, 260), mixer means (130, 135; 235) being provided to open said passage (160, 260) and to mix together the two substances before they are dispensed, and dispenser means (235, 135) being provided for dispensing said mixture, said mixer means comprising a first piston (135; 235) slidably received in one of the tanks (110; 210) to transfer one of the substances from its tank (110; 210) into the other tank (210; 110), said dispenser means including a second piston (235; 135) slidably received in the other tank (210; 110) for dispensing the mixture of the two substances via the outlet channel (120), the device being **characterized in that** said sealing means comprise at least one ball (115, 215) adapted to be expelled from the passage (160, 260) by the pressure created in one of the tanks (110; 210) when said first piston (135; 235) is actuated.

2. A device according to claim 1, in which said mixer means comprise a hollow rod (130) secured to the first piston (135) and adapted to penetrate into said passage (160, 260) to expel the balls (115, 215) and, after the two substances have been mixed together, extending to the second tank (210) in such a manner that said hollow rod (130) forms the inlet end of the outlet channel (120).

3. A device according to claim 1 or 2, in which said mixer means (130, 135; 235) are actuated independently of said dispenser means (235; 135).

4. A device according to any one of claims 1 to 3, in which said dispenser means (235; 135) are adapted to dispense the entire mixture of the two substances in a single actuation.

5. A device according to any one of claims 1 to 3, in which said dispenser means (235; 135) include or cooperate with stop means for subdividing the mixture of the two substances into a plurality of doses, one dose being dispensed on each actuation.

6. A device according to any preceding claim, in which the device comprises two independent subassemblies (100, 200), the first subassembly (100) having the outlet channel (120), the first tank (110), and one of the pistons (135), and the second subassembly (200) having the second tank (210) and the other piston (235), connection means (180, 280) being provided for connecting the two subassemblies (100, 200) together.

7. A device according to claim 6, in which each of the first and second tanks (110, 210) has a respective ball (115, 215) expelled during actuation of the mixer means.

8. A device according to any preceding claim, in which the mixer means comprise the piston (235) disposed in the second tank (210), actuation of said piston (235) creating sufficient pressure in the second tank (210) to expel the ball (115) from the passage (160) into the inside of the first tank (110) and to transfer the substance contained in the second tank (210) into the first tank (110) to mix therein with the other substance, the dispenser means including the piston (135) disposed in the first tank (110), with actuation thereof serving to dispense the mixture.

9. A device according to any preceding claim, in which one of the substances is a liquid and the other substance is a powder, mixing being performed extemporaneously by transferring the liquid into the tank containing the powder.

10. A device according to any preceding claim, in which said first tank (110) contains a solvent and said second tank (210) contains a powder, or vice versa.

11. A device according to claim 9 or 10, in which said powder is a pre-dosed liquid solution that has subsequently being freeze-dried.
